# EUROPEAN PATENT SPECIFICATION

(11) **EP 4 254 334 B1**
(45) Date of publication and mention of the grant of the patent: **25.11.2026**
(21) Application number: 23191949.9
(22) Date of filing: 20.05.2015
(51) Int. Cl.: G06T 7/60

(54) **SYSTEM AND METHOD FOR VALVE QUANTIFICATION**
SYSTEM UND VERFAHREN ZUR KLAPPENQUANTIFIZIERUNG
SYSTÈME ET PROCÉDÉ DE QUANTIFICATION DE VALVE

(30) Priority: 20.05.2014 US 201462001016 P
(43) Date of publication of application: 04.10.2023
(62) Document number(s) of the earlier application(s) in accordance with Art. 76 EPC: 15744993.5 / 3 146 507
(73) Proprietor: Materialise NV, 3001 Leuven (BE)
(72) Inventor: PIAZZA, Nicolo, Montreal Québec, H3A 1A1 (CA); VERSCHUEREN, Peter, 3001 Leuven (BE); PIETILA, Todd, Howell Michigan, 48843 (US)
(74) Representative: Arnold & Siedsma

(56) References cited:
- WO-A1-2010/067300
- US-A1- 2011 153 286
- US-A1- 2012 053 466
- ANDRES DIAZ LANTADA ET AL: "Development of Personalized Annuloplasty Rings: Combination of CT Images and CAD-CAM Tools", ANNALS OF BIOMEDICAL ENGINEERING, KLUWER ACADEMIC PUBLISHERS-PLENUM PUBLISHERS, NE, vol. 38, no. 2, 14 October 2009 (2009-10-14), pages 280 - 290, XP019765872, ISSN: 1573-9686
- BLANKE P ET AL: "Assessment of aortic annulus dimensions for Edwards SAPIEN Transapical Heart Valve implantation by computed tomography: calculating average diameter using a virtual ring method", EUROPEAN JOURNAL OF CARDIO-THORACIC SURGERY, SPRINGER VERLAG, BERLIN, DE, vol. 38, no. 6, 1 December 2010 (2010-12-01), pages 750 - 758, XP027527253, ISSN: 1010-7940, [retrieved on 20100515]
- J. ENDER ET AL: "Prediction of the annuloplasty ring size in patients undergoing mitral valve repair using real-time three-dimensional transoesophageal echocardiography", EUROPEAN JOURNAL OF ECHOCARDIOGRAPHY, vol. 12, no. 6, 4 May 2011 (2011-05-04), pages 445 - 453, XP055215858, ISSN: 1525-2167, DOI: 10.1093/ejechocard/jer042
- ENDER J ET AL: "Value of Augmented Reality-Enhanced Transesophageal Echocardiography (TEE) for Determining Optimal Annuloplasty Ring Size During Mitral Valve Repair", THE ANNALS OF THORACIC SURGERY, ELSEVIER, UNITED STATES, vol. 86, no. 5, 1 November 2008 (2008-11-01), pages 1473 - 1478, XP025560424, ISSN: 0003-4975, [retrieved on 20081017], DOI: 10.1016/J.ATHORACSUR.2008.07.073
- CHANDRAJIT BAJAJ ET AL: "Multi-component heart reconstruction from volumetric imaging", PROCEEDINGS OF THE 2008 ACM SYMPOSIUM ON SOLID AND PHYSICAL MODELING , SPM '08, 1 January 2008 (2008-01-01), New York, New York, USA, pages 193, XP055117373, ISBN: 978-1-60-558106-2, DOI: 10.1145/1364901.1364928
- RAMON CASERO ET AL: "Cardiac valve annulus manual segmentation using computer assisted visual feedback in three-dimensional image data", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 738 - 741, XP031883146, ISBN: 978-1-4244-4123-5, DOI: 10.1109/IEMBS.2010.5626303

## Description

### BACKGROUND OF THE INVENTION

### Field of the Invention

This application relates to quantification of space and volume of areas in a patient's anatomy. In some aspects, this application relates specifically to mitral valve quantification. Even more specifically, this application relates to a system and method for quantifying the mitral valve apparatus and its surroundings for use in selecting an appropriately sized valve in a catheter-based transcatheter mitral valve repair procedure.

### Description of the Related Technology

The human heart is a complex organ having many working parts which are critical to the proper functioning of the heart and the blood circulation and provides throughout the human body. The human heart is generally made up of four hollow chambers, the right atrium, the right ventricle, the left atrium, and the left ventricle. One of the keys to a properly functioning heart is the regulation of blood flow through these chambers. Regulation of blood throw through and between these chambers is provided by valves. For example, between the right atrium and the right ventricle, there is an atrioventricular opening.

The tricuspid valve is situated at that opening, and permits blood to move from the right atrium into the right ventricle. The valve opens when the blood pressure on the atrium site is greater than that on the ventricular side. When the valve opens, blood is permitted to flow from the right atrium into the right ventricle. When blood pressure is greater on the ventricle side, the valve closes. When the valve closes, blood is prevented from moving back in the other direction.

In the healthy heart, blood flow is also regulated between the left atrium and left ventricle. Here, the mitral valve allows blood to enter the left ventricle from the left atrium when the left atrium fills with blood and the pressure within the left atrium increases to a level above that of the left ventricle. When open, blood flows in a downward direction from the left atrium into the left ventricle, where it is pushed out to the rest of the body as part of the greater circulatory process. When a healthy mitral valve closes, blood flow between the two chambers is stopped, and this closing prevents a reversal of blood flow.

Unfortunately, mitral valves do not always function normally. An abnormally functioning mitral valve can lead to severe health problems. One abnormality associated with the mitral valve is mitral regurgitation ("MR"). Mitral regurgitation is a disorder in which the mitral valve does not close properly during contraction of the left ventricle. This causes blood that has passed from the left atrium into the left ventricle to reverse its flow back into the left atrium.

Mitral regurgitation may be treated surgically. One surgical option includes the replacement of the mitral valve where the mitral valve is replaced with either a bio prosthetic replacement or a synthetic replacement. Another surgical option includes repair of the mitral valve. Although mitral valve repair is generally seen as preferable to mitral valve replacement due to the less invasive nature of the procedure, at present, both options require open-heart surgery. Because many candidates for mitral valve replacement and repair are not good candidates for tolerating the stress of open-heart surgery, there has been ongoing research directed to developing transcatheter mitral valves. These transcatheter mitral valves can be introduced using a catheter-based system, obviating the need for a surgical procedure. Using noninvasive catheter-based implant techniques, the physical trauma associated with an open heart surgery may be minimized and more patients may be treated effectively for the mitral regurgitation disorder.

Although the use of transcatheter mitral valves shows great promise, there are significant challenges involved with effectively deploying these types of devices. In an open surgical procedure, the surgeon has complete access to the surgical site. Consequently, the surgeon is able to visually survey the site in order to perform the procedure effectively. When using the catheter-based system, however, the surgeon must rely and various imaging technologies to provide guidance to positioning and fit of a repair and/or replacement valve. Because transcatheter mitral valves are inserted using a delivery catheter, it is critical that the transcatheter mitral valve introduced into the patient have an appropriate size and shape to conform to the patient's anatomy. The need for appropriate sizing and shaping of implants extends well beyond mitral valve-related procedures, and may be useful in various different surgical contexts. At present, methodologies for quantifying the pertinent measurements of the mitral valve are inadequate. Accordingly, there is a need for a standardized measurement method which can be used for planning implantation of medical devices, including transcatheter mitral valve implantation.
Díaz Lantada, A., Valle-Fernández, R.D., Morgado, P.L. et al. Development of Personalized Annuloplasty Rings: Combination of CT Images and CAD-CAM Tools. Ann Biomed Eng 38, 280-290 (2010). https://doi.org/10.1007/s10439-009-9805-z. This paper discloses the use of personalized annuloplasty rings manufactured for each patient according to the size and morphology of their valve complex could be beneficial for the treatment of mitral insufficiency, this possibility has been limited for reasons of time-lines and costs as well as for design and manufacturing difficulties, as has been the case with other personalized implant and prosthetic developments. This paper sets out a personalized development of an annuloplasty ring based on the combined use of information from medical imaging, from CAD-CAM design programs and prototype manufacture using rapid prototyping technologies.
Blanke P, Siepe M, Reinöhl J, Zehender M, Beyersdorf F, Schlensak C, Langer M, Pache G. Assessment of aortic annulus dimensions for Edwards SAPIEN Transapical Heart Valve implantation by computed tomography: calculating average diameter using a virtual ring method. Eur J Cardiothorac Surg. 2010 Dec;38(6):750-8. doi: 10.1016/j.ejcts.2010.03.039. Epub 2010 May 15. PMID: 20472458. This paper discloses that an accurate preoperative assessment of the aortic annulus dimensions is critical in patients undergoing transcatheter aortic valve implantation (TAVI) for severe AS. Using multislice computed tomography (MSCT), a novel approach was evaluated to quantify aortic annulus dimensions using cross-sectional area (CSA) assessment and average diameter calculation compared with the commonly applied electronic caliper measurements in patients undergoing transapical implantation of the Edwards SAPIEN Transcatheter Heart Valve.
Ender J, Eibel S, Mukherjee C, Mathioudakis D, Borger MA, Jacobs S, Mohr FW, Falk V. Prediction of the annuloplasty ring size in patients undergoing mitral valve repair using real-time three-dimensional transoesophageal echocardiography. Eur J Echocardiogr. 2011 Jun;12(6):445-53. doi: 10.1093/ejechocard/jer042. Epub 2011 May 4. PMID: 21546375; PMCID: PMC3117468. This paper discloses to investigate the additional value of real-time three-dimensional transoesophageal echocardiography (RT 3D TOE)-guided sizing for predicting annuloplasty ring size during mitral valve repair.
Ender J, Koncar-Zeh J, Mukherjee C, Jacobs S, Borger MA, Viola C, Gessat M, Fassl J, Mohr FW, Falk V. Value of augmented reality-enhanced transesophageal echocardiography (TEE) for determining optimal annuloplasty ring size during mitral valve repair. Ann Thorac Surg. 2008 Nov;86(5):1473-8. doi: 10.1016/j.athoracsur.2008.07.073. PMID: 19049733. Herein it is disclosed that mitral valve (MV) annuloplasty is an integral part of MV repair, but sizing under direct vision is occasionally challenging. Furthermore, traditional sizing is not possible for percutaneous MV repair techniques. This study compared augmented reality-enhanced three-dimensional (3D) transesophageal echocardiography (TEE) for determining MV annuloplasty size with conventional surgical sizing. In patients undergoing elective MV repair, a 3D MV reconstruction was performed using TEE. Modified 4D valve assessment software was used to create 3D computer-aided design models of standard annuloplasty rings (28 to 36 mm), which were stored in a digital database. These virtual 3D annuloplasty ring templates were superimposed on the preoperative 3D TEE reconstructions of the MV, and results were compared with conventional sizing under direct vision.
US 20110153286 A1 discloses a method and system for virtual percutaneous valve implantation, wherein a patient-specific anatomical model of a heart valve is estimated based on 3D cardiac medical image data and an implant model representing a valve implant is virtually deployed into the patient-specific anatomical model of the heart valve. A library of implant models, each modeling geometrical properties of a corresponding valve implant, is maintained. The implant models maintained in the library are virtually deployed into the patient specific anatomical model of the heart valve to select an implant type and size and deployment location and orientation for percutaneous valve implantation.

### SUMMARY

In one embodiment, a method of method of mitral valve quantification is provided. The method may include generating a three-dimensional heart model and defining a three-dimensional mitral valve annulus. The method may further include fitting a plane through the three-dimensional mitral valve annulus. The distance between at least two papillary muscle heads may then be measured. The method further may include defining an average diameter of at least one cross section around the micro valve annulus. Based on the average diameter, a size of an implant may be selected.

In another embodiment, a computer-readable medium having computer-executable instructions stored thereon is provided. When the instructions are executed by a processor of a computing device, they cause the computing device to perform a method of mitral valve quantification. The method may include generating a three-dimensional ("3-D") model of a patient's heart from scanned images of the patient's heart and defining a 3-D mitral valve annulus. The method may also include fitting a plane through the 3-D mitral valve annulus. Distance may be measured between a first papillary muscle head and a second papillary muscle head in the 3-D model, and an average diameter of at least one cross section around the mitral valve annulus may be defined. The method may further include determining a size of an implant to be implanted.

### BRIEF DESCRIPTION OF THE DRAWINGS

Figure 1 is a block diagram of one example of a computing environment suitable for practicing various embodiments disclosed herein.
Figure 2 is a high-level system diagram of a computing system that may be used in accordance with one or more embodiments.
Figure 3 is a flowchart showing an example of a method of providing mitral valve quantification according to one or more embodiments.
Figure 4 is a flowchart of a sub process showing a more detailed view of the anatomical characterization of the mitral valve apparatus from Figure 3 according to one embodiments.
Figure 5 is a flowchart showing an example of a process by which a mitral valve annulus may be defined as described in the process shown in Figure 4.
Figure 6 is a flowchart providing a more detailed view of the visualization enhancement process from Figure 4 according to one or more embodiments.
Figure 7 is a flowchart providing additional detail about the distance measurement process described in Figure 4.
Figure 8A is a flowchart showing a sub-process for the vulnerable anatomical structure assessment referenced at block 310 in Figure 3.
Figure 8B is a flowchart showing an example of how the best fit size for an implant may be determined in accordance with one or more embodiments.
Figure 9 is an example of a graphical user interface environment which may be used to define the mitral valve annulus according to the process described in Figure 5.
Figures 10-12 provide examples of graphical user interfaces which can be used to calculate the 3-D surface area of the mitral valve annulus and fit a plane through the annulus according to aspects of the process described in Figure 4.
Figure 13 is a graphical illustration of open hollowed heart anatomy constructed according to aspects of the process described in Figure 6.
Figure 14 is the open hollowed heart anatomy from Figure 13 with various point-to-point measurements defined therein.
Figures 15 and 16 provide visual illustration of various aspects of the process described in connection with Figure 8A.
Figures 17-19C provide visual depictions of graphical user interface environments that may be used to simulate an implant using a primitive as described in connection with Figure 8B above.

### DETAILED DESCRIPTION OF CERTAIN INVENTIVE EMBODIMENTS

As noted above, determining the appropriate dimensions for an implanted device can play an important role in the success of an implantation procedure. In the context of the transcatheter mitral valve repair, appropriate dimensions and sizing of the device plays an important role in the success of a transcatheter mitral valve repair procedure. Recognizing the importance of mitral valve quantification, the inventors have devised systems and methods which quantify the mitral valve in three dimensions. The systems and methods typically provide for the calculation of a three-dimensional model of the blood volume in a patient's heart. From the three-dimensional model, a model of the myocardium and main anatomical structures in the heart can be also reconstructed. Using this reconstructed heart, a series of measurements may be performed from which a virtual insertion of a transcatheter mitral valve device can be simulated using three-dimensional computer modeling. Once the device has been virtually implanted within the three-dimensional heart model, the surgical site may be assessed by analyzing the potential impact of the device on anatomical structures could be harmed by physical features of the device. Based on that analysis, an appropriately sized mitral valve device can be selected for use in the repair procedure.

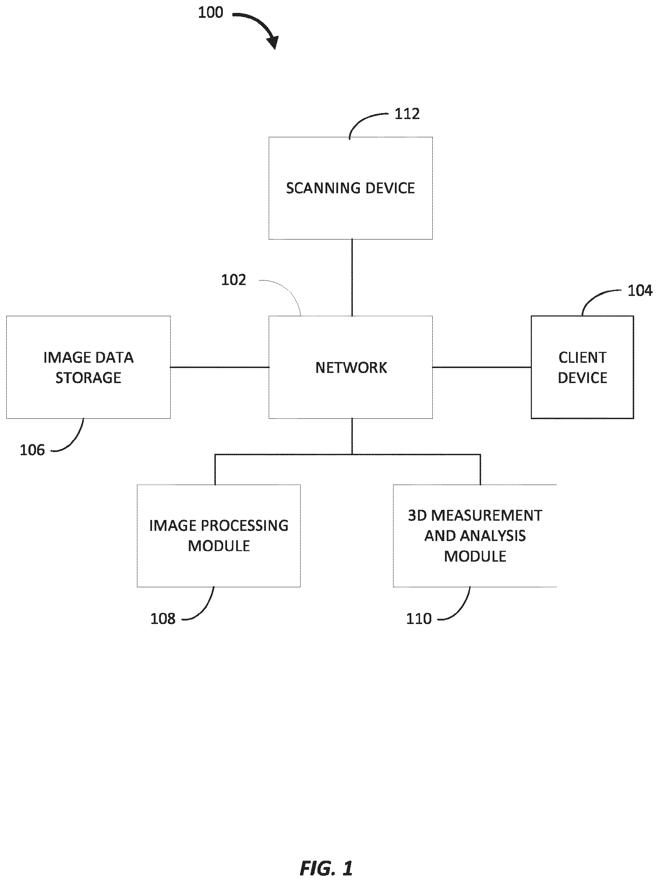

The systems and methods described herein may be implemented in a computing environment comprising one or more computing devices configured to provide various functionalities. Figure 1 is an example of a computer environment 100 suitable for implementing certain embodiments described herein. The computer environment 100 may include a network 102. The network 102 may take various forms. For example, the network 102 may be a local area network installed at a surgical site. In some embodiments, the network 102 may be a wide area network such as the Internet, for e in other embodiments, the network 102 may be a combination of local area networks and wide area networks. Typically, the network will allow for secured communications and data to be shared between various computing devices. Among these computing devices is a client device 104. The client device 104 may be a typical personal computer device that runs an off-the-shelf operating systems such as Windows, Mac OS, Linux, Chrome, or some other operating system. The client device 104 may have application software installed to allow it to interact via the network 102 with other software stored on various other modules and devices in the computing environment 100. This application software may take the form of a web browser capable of accessing a remote application service. Alternatively, the application software may be a client application installed in the operating system of the client device 104. Client device 104 may also take the form of a specialized computer, specifically designed medical imaging work, or even more specifically for mitral valve quantification. The client device 104 may further take the form of a mobile device or tablet computer configured to communicate via the network 102 and further configured to run one or more software modules to allow a user to perform various methods described herein.

The computer environment 100 may further include image data storage 106. Typically, the image data storage 106 takes the form of a large database designed to store image files captured by a scanning device 112. These images may be DICOM images, or other types of images. The image data storage 106 may be part of a scanning device 112, or alternatively it may be part of a client computing device 104. The image data storage 106 may also be in a standalone database having dedicated storage optimized for medical image data. The computer environment 100 may also include a scanning device 112. The scanning device 112 may typically is a medical imaging device which scans a patient to create images of their anatomy. In the computing environment 100 shown in Figure 1, the scanning device 112 may be a CT scanner or an MRI device. However, a skilled artisan will appreciate that other scanning technologies may be implemented which provide imaging data that can be used to create three-dimensional anatomical models.

As will be explained in detail below, the scanning device 112 may be configured to create cross-sectional images of a patient's heart. Those images may be stored in the image data storage 106, and utilized to create three-dimensional models of the heart. To that end, the computing environment 100 may also include an image processing module 108. The image processing module 108 may take the form of computer software, hardware, or a combination of both which retrieves the medical imaging data from image data storage 106 and generates a three-dimensional surface model using stacks of 2-D image data. The image processing module 108 may be a commercially available image processing software for three-dimensional design and modeling such as the Mimics application from Materialise NV. However, other image processing software may be used. In some embodiments, the image processing module 108 may be provided via a webbased network application that is accessed by a computer over the network (such as client device 104, for example). Alternatively, the image processing module may be a software application that is installed directly on the client device 104, and accesses image data storage 106 via the network 102. In general, the image processing module 108 may be any combination of software and/or hardware located within the computing environment 100 which provides image processing capabilities on the image data stored within the image data storage 106.

The computing environment also may include a three-dimensional measurement and analysis module 110 ("3-D measurement and analysis module"). The 3-D measurement and analysis module 110 may be software that is complementary to and/or bundled with the image processing module 108. For example, the 3-D measurement and analysis module 110 may be a bundled projects such as 3matic^{®} from Materialise NV. The 3-D measurement and analysis module may also take the form of general CAD and design software such as, for example, AutoCAD or SolidWorks. In some embodiments, the 3-D measurement and analysis module may be a specialized application created specifically for mitral valve quantification purposes. As will be explained in further detail below, the 3-D measurement and analysis module 110 will be generally used to determine precise measurements of various aspects of the patient anatomy in order to determine the appropriate dimensions for a surgical implant. In particular examples provided below, the heart anatomy is measured and in order to determine appropriate dimensions for a transcatheter mitral valve implant. As with the image processing module 108, the 3-D measurement and analysis module 110 may be a network-based application which is accessed via a web browser by one or more client devices 104. It may also be a native application installed into the operating system of a computer such as, client device 104 for example. In still other embodiments, the 3-D measurement and analysis module 110 may be a network application which is run as a client/server implementation.

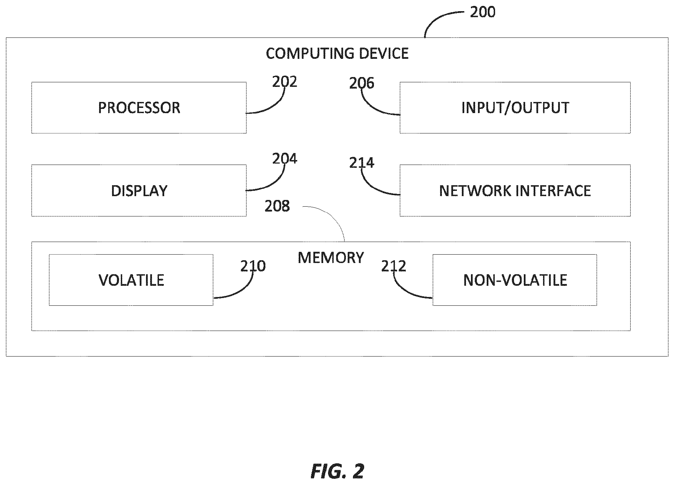

Various embodiments of the invention may be implemented using general and/or special purpose computing devices. Turning now to Figure 2, an example of a computing device 200 suitable for implementing various embodiments of the invention is shown. The computer system 200 may generally take the form of computer hardware configured to execute certain processes and instructions in accordance with various aspects of one or more embodiments described herein. The computer hardware may be a single computer or it may be multiple computers configured to work together. The computing device 200 includes a processor 202. The processor 202 may be one or more standard personal computer processor such as those designed and/or distributed by Intel, Advanced Micro Devices, Apple, ARM, or Motorola. The processor 202 may also be a more specialized processor designed specifically for image processing and/or analysis. The computing device 200 may also include a display 204. The display 204 may be a standard computer monitor such as, an LCD monitor as is well known. The display 204 may also take the form of a display integrated into the body of the computing device, for example as with an all-in-one computing device or a tablet computer.

The computing device 200 may also include input/output devices 206. These may include standard peripherals such as keyboards, mice, printers, and other basic I/O software and hardware. The computing device 200 may further include memory 208. The memory 208 may take various forms. For example, the memory 208 may include volatile memory 210. The volatile memory 210 may be some form of random-access memory, and may be generally configured to load executable software modules into memory so that the software modules may be executed by the processor 202 in a manner well known in the art. There the software modules may be stored in a nonvolatile memory 212. The non-volatile memory 212 may take the form of a hard disk drive, a flash memory, a solid-state hard drive or some other form of non-volatile memory. The nonvolatile memory 104B may also be used to store non-executable data, such database files and the like.

The computer device 200 also may include a network interface 214. The network interface may take the form of a network interface card and its corresponding software drivers and/or firmware configured to provide the system 200 with access to a network (such as the Internet, for example). The network interface card 214 may be configured to access various different types of networks, such as those described above in connection with Figure 1. For example the network interface card 214 may be configured to access private networks that are not publicly accessible. The network interface card 214 may also be configured to access wireless networks such using wireless data transfer technologies such as EVDO, WiMax, or LTE network. Although a single network interface 214 is shown in Figure 2, multiple network interface cards 214 may be present in order to access different types of networks. In addition, a single network interface card 214 may be configured to allow access to multiple different types of networks.

In general, the computing environment 100 shown in Figure 1 may generally include one, a few, or many different types of computing devices 200 which work together to carry out various embodiments described below. A skilled artisan will readily appreciate that various different types of computing devices and network configurations may be implemented to carry out the inventive systems and methods disclosed herein.

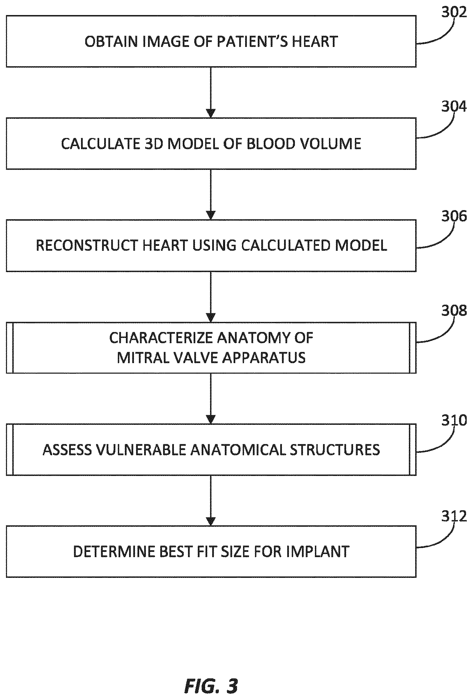

Figure 3 is a high-level flow diagram showing a process by which quantification of space and/or volume of a particular area of a patient's anatomy, in this example the mitral valve, may be realized according to one or more embodiments. Process begins at block 302, wherein image of the patient's heart acquired. The image may be acquired using the scanning device 112 shown in Figure 1, such as a CT scanner or an MRI machine. In acquiring the image, a contrast agent may be used in order to improve the visibility of various internal structures of the heart. The image (or images) acquired using the scanning device 112 may be stored in image data storage 106 or some other computer memory accessible via the computer network 102. The process then moves to block 304. There a 3-D model of blood volume is calculated based on the acquired image. The use of a contrast agent in the previous step allows for the 3-D modeling of the blood volume. The 3-D model may be calculated using the image processing module 108, or some other software and/or hardware designed to generate 3-D models from CT and/or MRI image data.

Using the 3-D model of the blood volume, the anatomical structures of the heart may be reconstructed at block 306. Typically, it is easier to first obtain the blood volume from the clearly visible contrast agent and then use that to create the myocardium. However, a skilled artisan will appreciate that it is also possible to directly create the myocardial model. This reconstruction may also be performed using the image processing module 108. The reconstruction of the heart anatomy typically begins with segmentation of the left side of the heart, followed by optimization of the segmented and reconstructed 3-D model using optimization tools such as wrapping functions and smoothing functions to clean the surfaces of the models.

The process next moves to block 308. There, the anatomy of the mitral valve apparatus is characterized by defining control points and taking measurements of relevant anatomical structures. Typically, these measurements are performed using the 3-D measurement and analysis module 110. As discussed above, this 3-D measurement and analysis module 110 may be software that is bundled or even integrated with the image processing module 108. These measurements may include various steps. For example, using the 3-D measurement and analysis module 110, control points may be placed on the 3-D model of the heart which define the mitral valve annulus. These control points may be defined using a spline drawing function provided by the 3-D measurement and analysis module 110.

With the mitral valve annulus defined, the measurements may further include calculation of the 3-D surface area of the mitral valve annulus based on the spline. Additional measurements and analysis may be performed relating to the mitral valve annulus. For example, and as will be discussed below, one or more planes can be fit through the mitral valve annulus using the 3-D measurement and analysis module. Using these defined planes, additional measurements can be taken which help to more precisely define the actual geometry of the mitral valve annulus.

Once the measurements have been taken, the process then moves to block 310. There an assessment is made of vulnerable anatomical structures. An anatomical structure may be vulnerable because a transcatheter mitral valve implant will not typically be fixed within the mitral valve. Rather, the mitral valve apparatus will experience a high degree of mobility. This movement can potentially damage surrounding anatomical structures from either collisions or protrusions from the device. This assessment may be made based on the measurements taken in the previous step. Moreover, it may further be a visual assessment. Finally, at block 312, based on the measurements taken and the assessed vulnerabilities, and appropriately sized mitral valve device may then be selected.

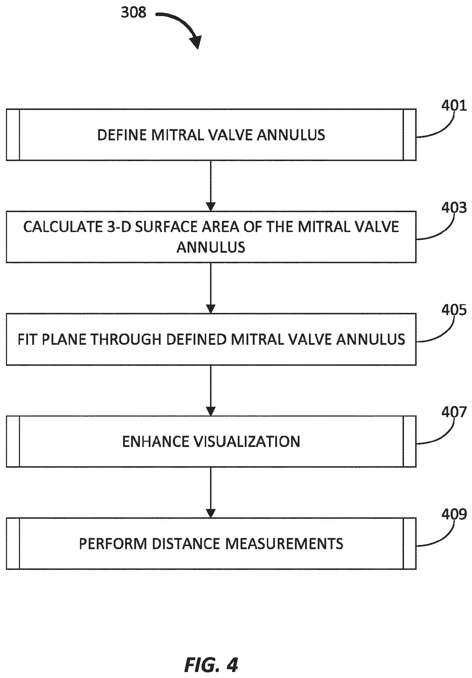

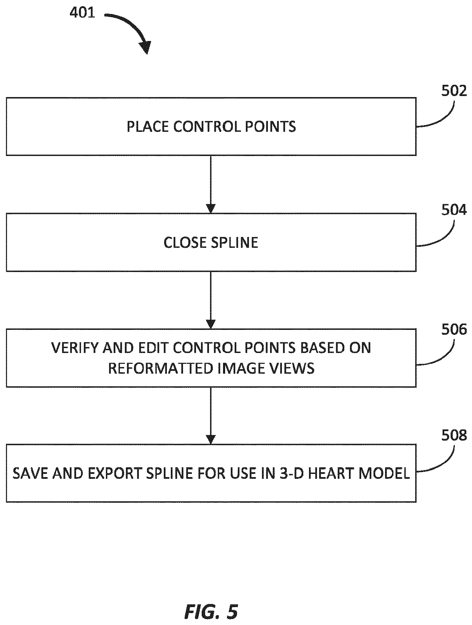

As discussed above in connection with block 308 of Figure 3, in certain embodiments, the mitral valve quantification process may include characterizing the anatomy of the mitral valve apparatus. Figure 4 is a flow diagram providing one example of a sub process which may be implemented to characterize the anatomy is provided in block 308. The sub process begins at block 401 where, using the image processing module, the mitral valve annulus is defined within the reconstructed heart model. Additional details about how the mitral valve annulus is defined will be discussed in connection with Figure 5 below. Once the mitral valve annulus has been defined, the process moves to block 403. There, the 3-D surface area of the mitral valve annulus is calculated. The process next moves to block 405 where a plane is fitted through the mitral valve annulus. In some embodiments, the plane may be generated by using a create datum plane function provided by the image processing module 108 and/or the 3-D measurement and analysis module 110. In fitting the plane through the mitral valve annulus, the 3-D surface area of the mitral valve annulus (the mitral surface) may be used as the fitting entity.

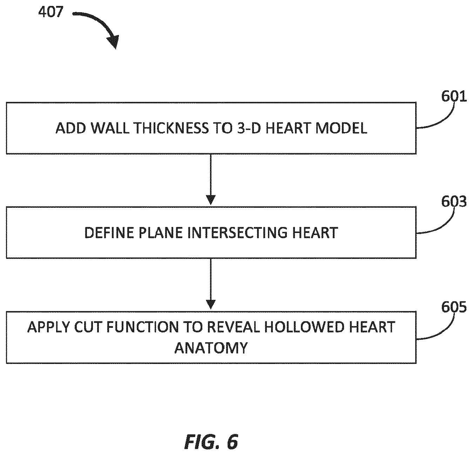

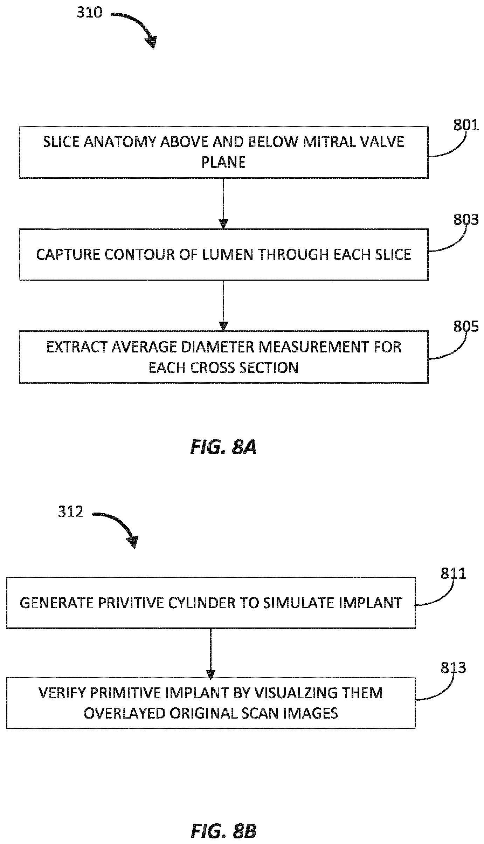

Next, the process moves to block 407. Here, the visualization of the 3-D heart model is enhanced by adding additional anatomical detail to the model and generating a view of a hollowed heart anatomy. Additional details about this enhancement of the visualization are provided below in connection with Figure 6. Once the visualization of the 3-D model has been enhanced, the process then moves to block 409. At block 409, distance measurements are performed on the model in order to determine various dimensional attributes that may impact the size of the transcatheter mitral valve implant. These distance measurements may be performed against various aspects of the 3-D model, and are discussed in further detail below in connection with Figure 8.

As indicated above, Figure 5 is a flow diagram which provides additional details about the process of defining the mitral valve annulus in the 3-D model. In this particular example, the mitral valve annulus may be defined by first placing control points on a user interface which graphically displays the 3-D model of the heart to the user. The control points may be placed using a spline drawing function provided by the 3-D measurement and analysis module 110. Once each of the control points for the defined mitral valve annulus has been selected, the initial control point may then be selected to close the spline at block 504. Once the spline has been closed, the process may then move to block 506 where the control points may be verified and edited based on reformatted image views generated from the data initially required by the scanning device 112. For example, the control points selected by the user may be superimposed on each of a coronal reformation, a sagittal reformation, as well as a conventional axial view. If the control points are inconsistent with the anatomy shown in any of the reformatted image views, they may be edited to ensure that they are consistent with the originally acquired image data. If the control points are consistent with the anatomy shown in the reformatted image views, then the process then may move to block 508 where the spline object is saved and exported for use in the 3-D heart model.

Turning now to Figure 6, a more detailed flow diagram provides additional details about the visualization enhancement of block 407 and Figure 4. In particular, Figure 6 provides an example of one implementation of the visual enhancement process according to one embodiment. The process begins at block 601, where a wall thickness is added to the 3-D heart model. Because the original 3-D heart model was generated using images based on blood volume, those images do not account for wall thickness in the model. In some embodiments, the wall thickness may be added by applying a hollow operation to the heart model and specifying a wall thickness as part of that operation. Once the wall thickness has been added, the process may then move to block 603 where a plane intersecting the heart may be defined. In some embodiments, the plane may be used to section the anatomy to provide an internal view of the geometry of the left heart side. In one embodiment, a three-point method may be implemented to define the plane which intersects the septum and the ascending aorta. However, a skilled artisan will appreciate that the precise location of the points can be modified, and that a skilled artisan, will appreciate however, that other methods of obtaining a view of the internal geometry may be utilized. Once the plane intersecting the heart has been defined, a cut function may then be applied to the hollowed heart anatomy at block 605. In applying the cut function to the hollowed heart anatomy, the defined plane on the septum may be used as the cutting entity. Applying the cut function results in a cutaway view of the interior of the heart anatomy.

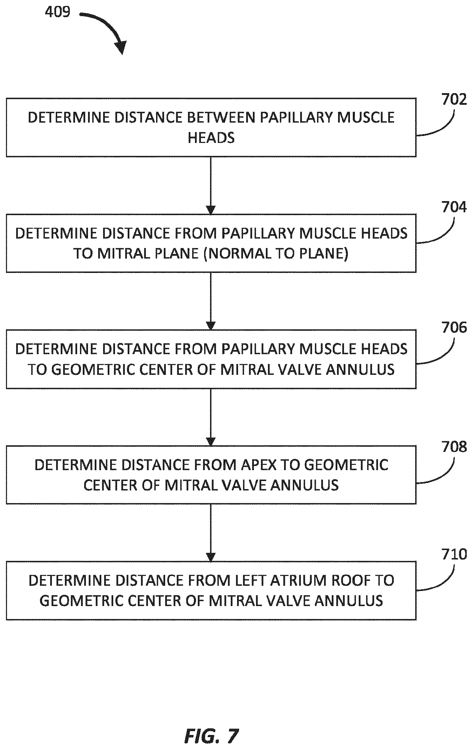

Figure 7 is a more detailed flowchart of the distance measurement step shown in block 409 of Figure 4. In this example, various distance measurements may be taken using the 3-D measurement and analysis module 110. In this particular example, the process begins at block 702 where the distance between papillary muscle heads is determined. Utilizing the 3-D measurement and analysis module 110, a user may identify and select two points in the cutaway view generated by the cut function applied in Figure 6. These two selected points may correspond to each of the papillary muscle heads. The 3-D measurement and analysis module 110 may, based on the location of these two points, determine the distance between the papillary muscle heads.

Next, the process moves to block 704. There the distance from each of the papillary muscle heads to the mitral plane is calculated. In this particular example the distance is calculated normal to the mitral plane, and not at the geometric center of the plane. Additional measurements may also be performed. The process next they move to block 706 where the distance from the papillary muscle heads to the geometric center of the mitral valve annulus is calculated. Next the process moves to block 708 where the distance from the apex of the heart to the geometric center of the mitral valve annulus is determined. The process then moves to block 710, where the distance from the left atrium roof to the geometric center of the mitral valve annulus is calculated.

These distance measurements can be used to select an implant design which avoids collisions with various anatomical structures after implantation. A transcatheter mitral valve implant may include various metallic components. When implanted into the mitral valve annulus, it takes a specific height and shape. The height and shape of the implanted device may interact substantially with several anatomical structures within the patient. For example, on the atrial side, the implant could collide with touch and possibly damage the thin walls of the atrium. In particular, the contours of the implant could potentially puncture the atrial wall because of its high mobility and deformation between systole vs diastole. In addition, on the ventricular side the deformations are also quite large, and the papillary muscles can interfere with the valves on the device by pushing against the frame of the valve. Additionally, they may even interfere with the new leaflets which are sewn into the metallic structure. This would mean the new valve would not be able to close or might even wear out faster because of this mechanical and repeatable contact. Accordingly, the distance measurements may be analyzed to account for the mobility of the device and select a size which will avoid these problems.

Turning now to Figure 8A, a more detailed flowchart is provided which provides an example of a detailed process for assessing vulnerable anatomical structures shown in Figure 3. This sub-process begins at block 801. There using the 3-D measurement and analysis module 110, a user may slice the anatomy both above and below the mitral valve plane. In some embodiments, the translate function may be used to copy the mitral valve plane above and below the mitral valve annulus. In one specific implementation the plane may be translated at 5 mm increments up to 20 mm.

The process that moves to block 803 where the contour of the lumen (e.g., the mitral valve) is captured through each slice created using the translate function. In some embodiments, an intersection curve may be calculated between the blood volume anatomy provided by a 3-D model and each plane in order to capture the contour of the lumen through each slice. The process next moves to block 805, where the system may extract the average diameter measurements at each of the cross sections. In one embodiment, the average diameter measurements may be extracted using an arc method or some other function that can be used to create an arc or curve.

As discussed above in connection with Figure 3, after vulnerable anatomical structures have been assessed, the size fit for a transcatheter mitral valve implant is determined. Figure 8B is an example of a process by which the best size fit can be selected according to one or more embodiments. The process begins at block 811, where a primitive cylinder may be generated to simulate the implant. The primitive cylinder may be generated based indirectly on the measurements determined in connection with Figure 7, and also more directly based on the captured contour of the lumen of the mitral valve annulus obtained using the process described in connection with Figure 8A above. Once the primitive cylinder has been created, the process then moves to block 813 where the primitive cylinder implant is verified by visualizing the contours of the objects overlaid on the original scanned images. Although the primitive generated in this example is a cylinder, a skilled artisan will readily appreciate that any number of other primitives may also be used separately or in combination with the cylinder.

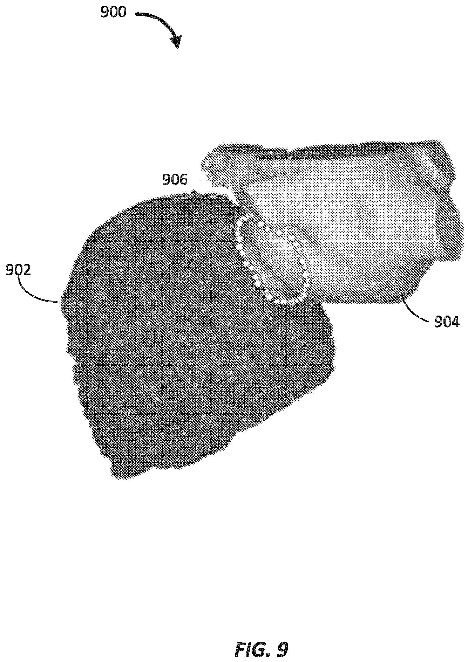

Although the general process described in connection with Figure 3 and the subprocesses described in connection with Figures 4-8B may be performed using various different configurations of computer hardware and/or software, Figures 9-14 provide examples of graphical user interfaces and computer generated images which may be utilized in performing the process described above.

Turning now to Figure 9, an example of a graphical user interface environment which may be used to define the mitral valve annulus as described above in connection Figure 5. As shown, a 3-D surface model of the left side of the patient's heart is provided. Superimposed onto the 3-D surface model of the heart 900 is a spline 906 created using a spline drawing function provided by the image processing module 108. The spline 406 defines the mitral valve annulus and is created by the user inputting the control points shown enclosing the spline by selecting the initial control point.

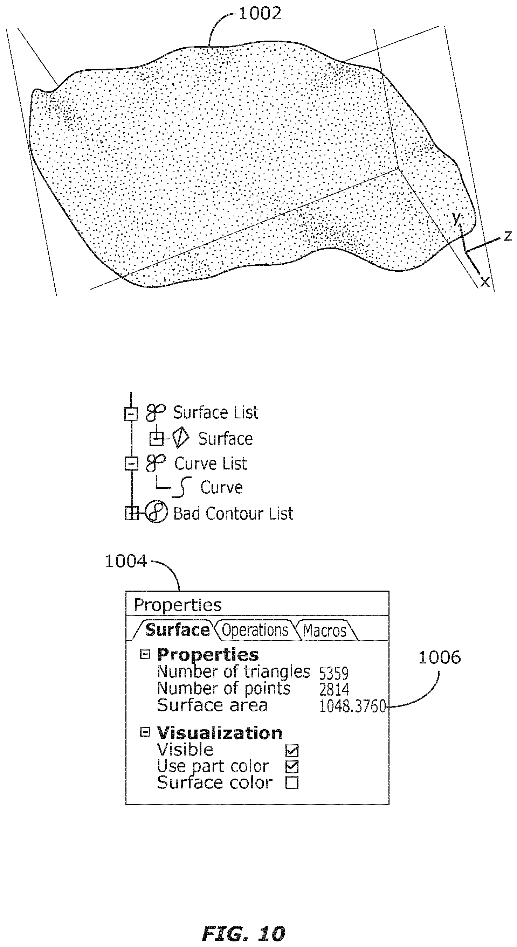

Figures 10-12 is an example of graphical user interface environment which can be used to calculate the 3-D surface area of the mitral valve annulus and fit a plane through the annulus as described above in blocks 403 and 405 of Figure 4. Figure 10 illustrates how the 3-D surface area of the mitral valve annulus is displayed. As shown the surface area 1002 of the mitral valve annulus is referenced and various properties 1004, including the calculated surface area 1006 are shown. The calculated surface area 1006 may be used to calculate the size of the implant device. In addition to the calculated surface area, the annulus projected circumference or a combination of other measurements may also govern the ultimate best fitting device.

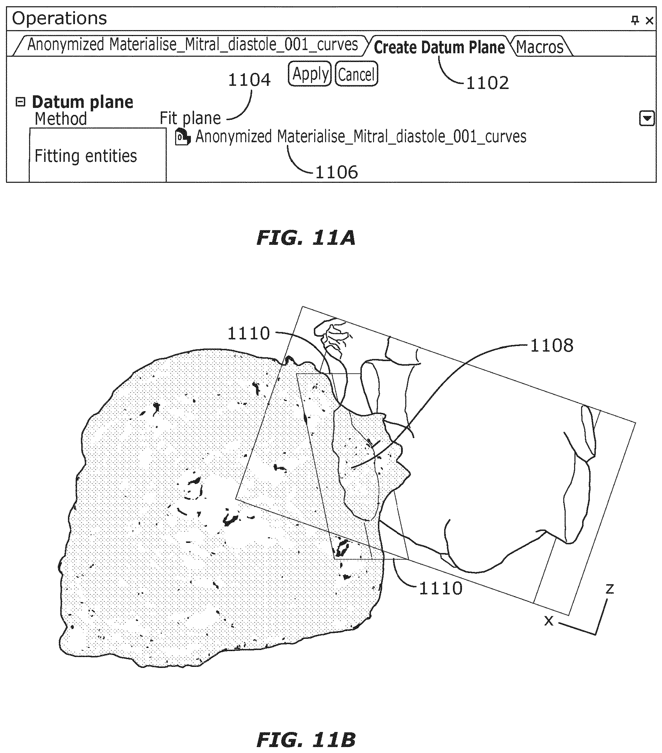

Figures 11A and 11B provide graphical depictions of aspects of a user interface by which a plane may be fit through the mitral annulus using a create datum plane function. As shown, a create datum plane operation 1102 has been selected. A fit plane method 1104 for creating the datum plane has been selected, and the fitting entity 1106 has been chosen. In this particular case, the mitral surface from Figure 10 has been selected as the fitting entity. Figure 11B provides a graphical illustration of how the plane is fit onto the 3-D heart model. As shown, a plane has been fit through the mitral annulus, with the geometric center 1108 of the mitral valve annulus as the origin of the fit plane 1110.

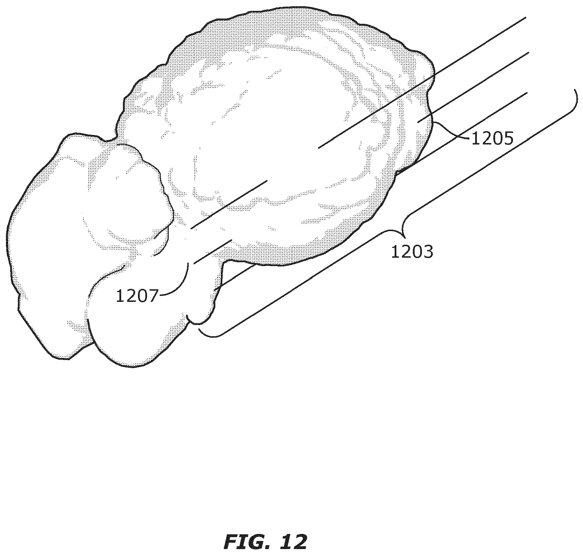

As discussed above in connection with Figure 6, wall thickness may be added to the 3-D heart model, and a plane may be defined intersecting the heart which can then be cut to reveal a hollowed heart anatomy. Turning now to Figure 12, a graphical illustration is provided showing how the intersecting plane may be defined. Here, the intersecting plane 1203 is defined using a three-point method which intersects the inter-ventricular septum 1205 (separating the left ventricle from the right ventricle) and ascending aorta 1207.

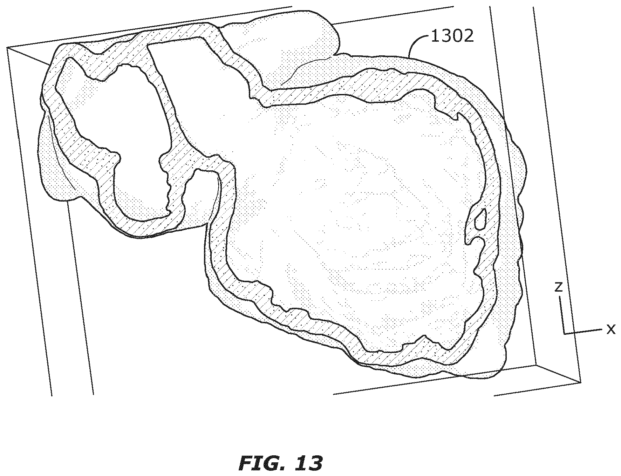

Figure 13 provides a cutaway view 1302 of the 3-D heart model after the cut function has been applied to the hollowed heart anatomy as described at block 605 of Figure 6. As discussed in connection with Figure 6, in this particular example the defined plane shown in Figure 12 has been used as the cutting entity.

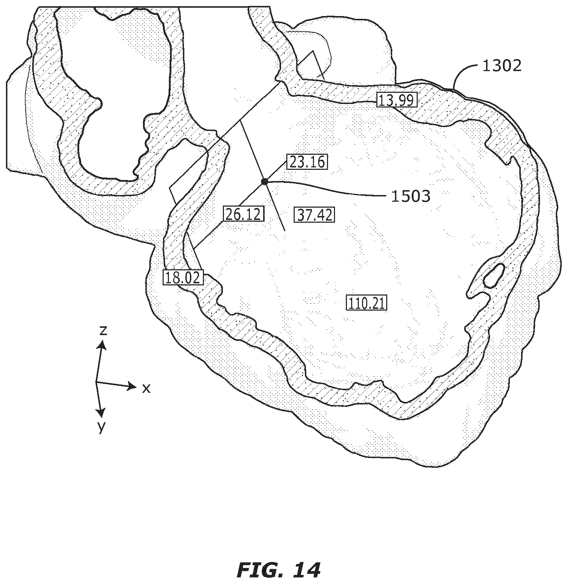

Figure 14 provides a visual illustration of how the various measurements described in Figure 7 may be carried out within a graphical user interface environment. Figure 14 shows the cutaway view 1302 from Figure 13. The cutaway view 1302 shows various measurements that have been extracted using a point-to-point measurement tool. Various measurements are shown with respect to the geometric center 1403 of the mitral annulus. Other measurements include the distance between the papillary muscle heads (37.42 mm), distance from the papillary muscle heads to the mitral plane (18.02 mm and 13.99 mm), the distance from the papillary muscle heads to the geometric center of the mitral valve annulus (26.12 mm and 23.16 mm), and the distance from the apex of the left ventricle to the geometric center of the mitral valve annulus (110.21 mm).

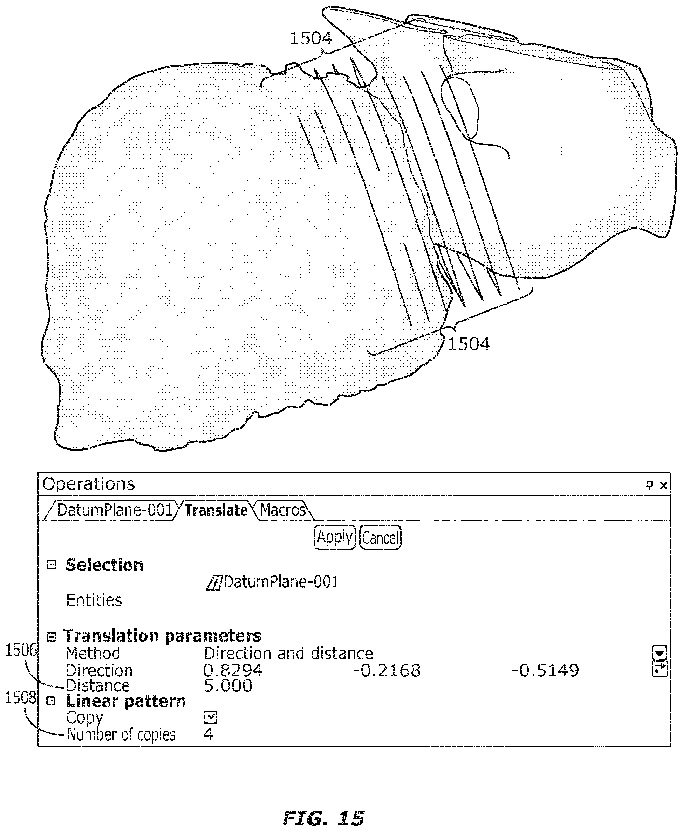

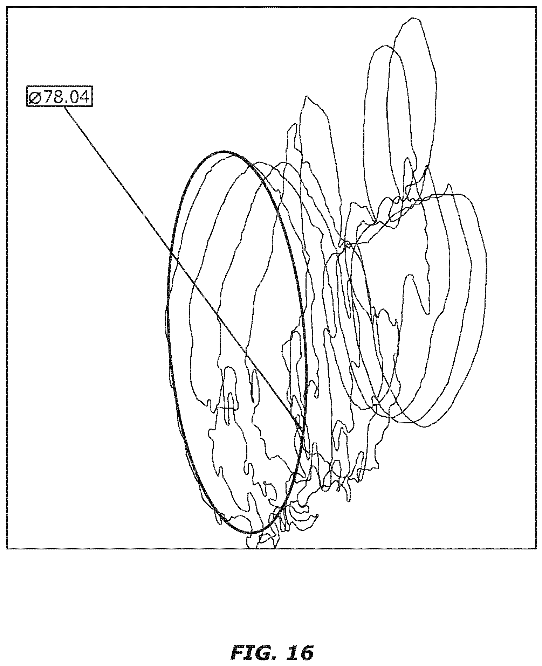

Turning now to Figures 15 and 16, a visual illustration of the process described in Figure 8A is provided. As shown, a Translate function three measurement and analysis module 110 has been used to copy the mitral valve plane 1504 above and below the annulus. The plane is translated at 5 mm increments to 20 mm as reflected by the values in the distance field 1506 and the number of copies field 1508. As noted above in Figure 8A, an intersection curve may be calculated between the blood volume anatomy 302 and each plane 1504, thereby capturing the contour of the lumen through each slice. Turning now to Figure 16, a graphical illustration is shown depicting how the average diameter measurement is extracted for each cross-section. As discussed above, these measurements may be extracted at these cross sections using an arc method.

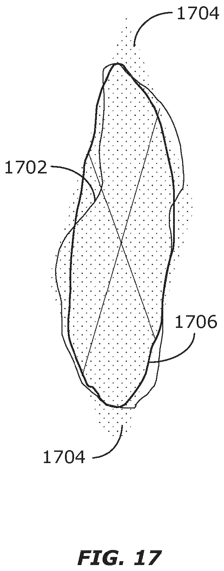

Figure 17 provides a visual illustration of one graphical user interface environment that may be used to simulate an implant using a primitive cylinder as described in connection with Figure 8B above. As shown, the mitral annulus curve 1702 is projected into the plane 1704 fit through the mitral annulus. The mitral annulus curve has been exported resulting in a flattened annulus 1706 and the diameter of the flattened annulus may be measured and used to size the primitive implant.

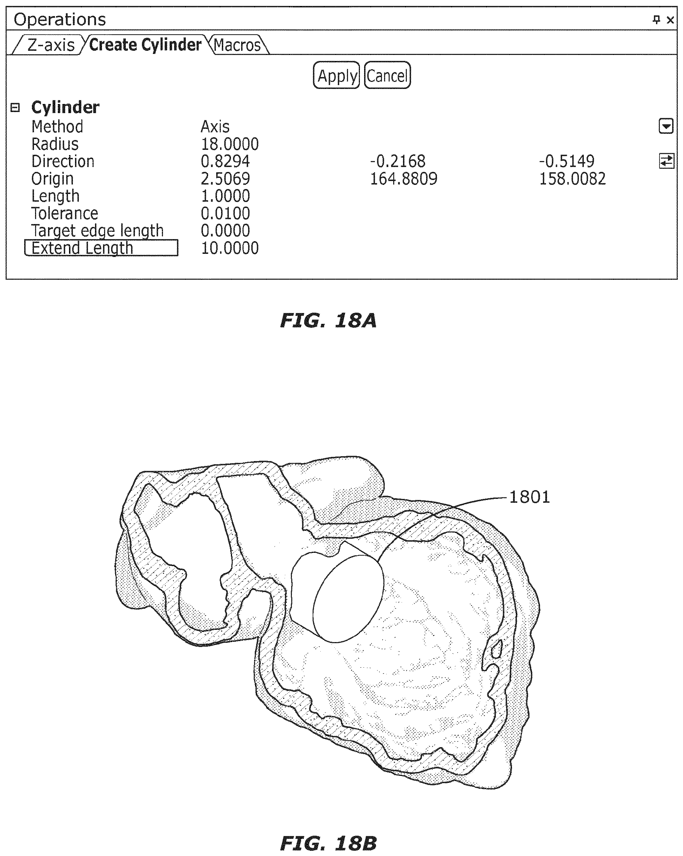

Figures 18A and 18B provide examples of a graphical user interface which may be used to create a simulated implant using a primitive cylinder. As shown in Figure 18A, the create cylinder operation in the 3-D measurement and analysis module 110 is applied using the measurements obtained above. Figure 18B shows the generated cylinder 1801 positioned within the mitral valve annulus.

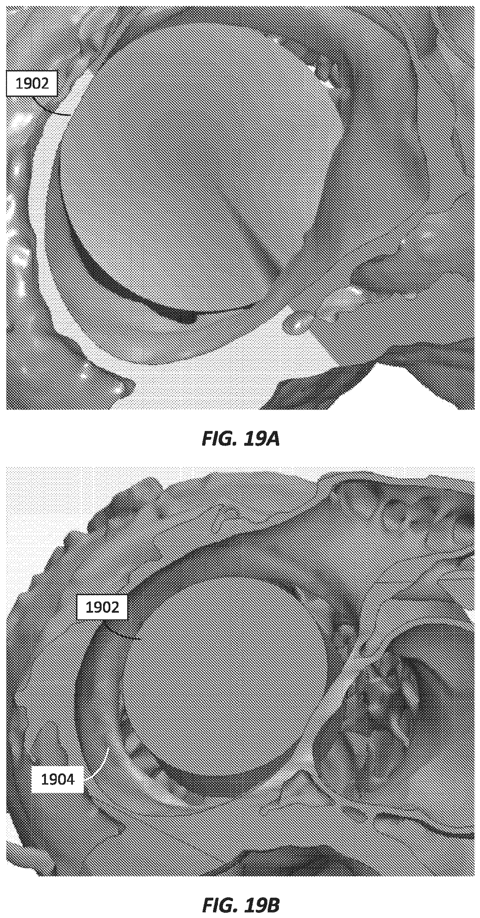

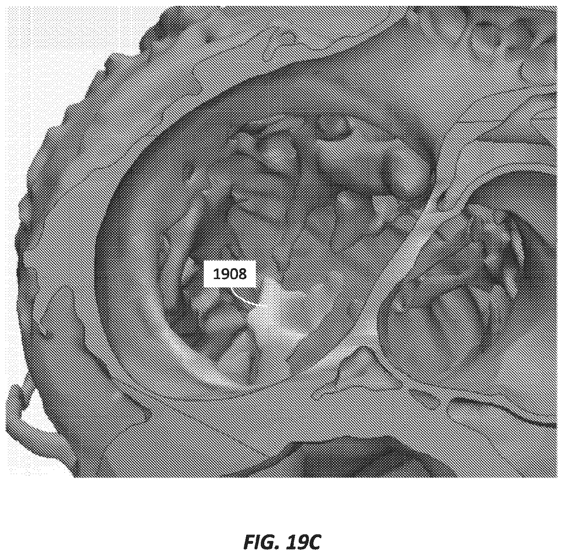

In some embodiments, the cylinder or some other type of geometry (e.g., primitive shape, CAD or scan file of the device as above) may be "virtually" implanted as shown in Figure 19A. Here, a distance mapping may be performed with respect to the anatomy. This distance mapping may quantify the space that is available between the implanted device and each relevant item of anatomy. As shown a primitive 1902 (which in this example is a cylinder, but may take various other forms) has been virtually placed within the mitral valve annulus. Turning to Figure 19B, a distance mapping is used to identify anatomical the position of anatomical structures in the heart (such as the walls of the atrium) or other anatomical features 1908 as shown in Figure 19C. The distance mapping provides a clear visualization of the spacing between the proposed implant and the anatomy.

Using the systems and methods described above, a standardized method provides physicians and researchers the ability to quantify the mitral valve apparatus and its surroundings for transcatheter mitral valve repair research and development as well as determining the appropriate sizing in the context of patient and procedure planning. Although the particular examples above relate to quantification of the mitral valve, a skilled artisan will appreciate that the principles, systems, and methods described above can be readily applied in connection with other types of surgical procedures and other areas of the anatomy. For example, in some embodiments, the valve may be a pulmonary branch valve. In other embodiments, the systems and methods described above may be used in the treatment of pulmonary artery stenosis. In other implementations, measurement and quantification of holes resulting from congenital heart defects, such as atrial septal defects ("ASDs") or ventricular septal defects (VSDs) may also be performed to select an appropriate size for a catheter implant or other device for implantation.

It is to be understood that any feature described in relation to any one embodiment may be used alone, or in combination with other features described, and may also be used in combination with one or more features of any other of the embodiments, or any combination of any other of the embodiments.
As further examples, reference is made to the following embodiments:
1. A method of quantifying a valve in an organ, the method comprising:
   generating a three-dimensional ("3-D") model of the organ from scanned images of the organ;
   defining a 3-D valve annulus;
   fitting a plane through the valve annulus;
   measuring distance between a first location and a second location in the 3-D model;
   defining an average diameter of at least one cross section around the valve annulus; and
   determining a size of an implant to be implanted.
2. The method of Embodiment 1, wherein the valve is a mitral valve, and wherein the organ is a patient's heart, and wherein measuring distance between the first location and the second location comprises measuring distance between a first papillary muscle head and a second papillary muscle head in the 3-D model.
3. The method of Embodiment 2, wherein generating the 3-D model comprises:
   obtaining an image of the patient's heart;
   calculating a 3-D model of blood volume; and
   reconstructing the patient's heart using the calculated model.
4. The method of Embodiment 2, wherein defining the mitral valve annulus comprises:
   placing control points on the 3-D heart model, wherein the control points define the mitral valve annulus;
   verifying the control points using reformatted image views of the patient's heart; and
   exporting the control points for use in the 3-D model.
5. The method of Embodiment 4, wherein the control points comprise a spline, and wherein the spline is defined by selecting the control points.
6. The method of Embodiment 2, wherein measuring distance between the first papillary muscle head and the second papillary muscle head comprises:
   adding wall thickness to the 3-D model;
   defining a plane intersecting a septum and an ascending aorta of the patient's heart;
   applying a cut function to reveal a cutaway view of the patient's heart anatomy;
   performing the measuring using a point-to-point measurement tool,
7. The method of Embodiment 2, further comprising:
   measuring distance from at least one papillary muscle head to the fitted plane;
   measuring distance from at least one papillary muscle head to a geometric center of the defined mitral valve annulus;
   measuring distance from a ventricular apex of the patient's heart to the geometric center of the defined mitral valve annulus; and
   measuring distance from a left atrium roof to the geometric center of the defined mitral valve annulus.
8. The method of Embodiment 2, wherein determining a size of an implant to be implanted comprises:
   generating a primitive to simulate the implant; and
   verifying the cylinder by visualizing the generated cylinder overlaid the scanned images of the patient's heart.
9. The method of Embodiment 8, wherein the scanned images of the patient's heart are at least one of CT images and MRI images.
10. The method of Embodiment 2, wherein defining an average diameter of at least one cross section around the mitral valve annulus comprises:
   defining a mitral valve plane;
   slicing anatomy above and below the defined mitral valve plane;
   capturing a contour of lumen through each cross-section; and
   extracting average measurements for each cross-section.
11. The method of Embodiment 10, wherein the average measurements are extracted using at least one of an arc method, a circle fit, an elliptical fit, and a freeform line segment fit.
12. The method of Embodiment 1, wherein the valve is a pulmonary valve.
13. A computer-readable medium having computer-executable instructions stored thereon, which, when executed by a processor of a computing device, cause the computing device to perform a method of mitral valve quantification, the method comprising:
   generating a three-dimensional ("3-D") model of the organ from scanned images of the organ;
   defining a 3-D valve annulus;
   fitting a plane through the valve annulus;
   measuring distance between a first location and a second location in the 3-D model;
   defining an average diameter of at least one cross section around the valve annulus; and
   determining a size of an implant to be implanted.
14. The computer-readable medium of Embodiment 13, wherein the valve is a mitral valve, and wherein the organ is a patient's heart, and wherein measuring distance between the first location and the second location comprises measuring distance between a first papillary muscle head and a second papillary muscle head in the 3-D model.
15. The computer-readable medium of Embodiment 14, wherein generating the 3-D model comprises:
   obtaining an image of the patient's heart;
   calculating a 3-D model of blood volume; and
   reconstructing the patient's heart using the calculated model.
16. The computer-readable medium of Embodiment 14, wherein defining the mitral valve annulus comprises:
   placing control points on the 3-D heart model, wherein the control points define the mitral valve annulus;
   verifying the control points using reformatted image views of the patient's heart; and
   exporting the control points for use in the 3-D model.
17. The computer-readable medium of Embodiment 16, wherein the control points comprise a spline, and wherein the spline is defined by selecting the control points.
18. The computer-readable medium of Embodiment 14, wherein measuring distance between the first papillary muscle head and the second papillary muscle head comprises:
   adding wall thickness to the 3-D model;
   visualizing the inside of the heart's anatomy;
   performing the measuring using a point-to-point measurement tool.
19. The computer-readable medium of Embodiment 14, further comprising:
   measuring distance from at least one papillary muscle head to be fitted plane;
   measuring distance from at least one papillary muscle head to a geometric center of the defined mitral valve annulus;
   measuring distance from a ventricular apex of the patient's heart to the geometric center of the defined mitral valve annulus; and
   measuring distance from a left atrium roof to the geometric center of the defined mitral valve annulus.
20. The computer-readable medium of Embodiment 14, wherein determining a size of an implant to be implanted comprises:
   generating a primitive cylinder to simulate the implant; and
   verifying the primitive cylinder by visualizing the generated primitive cylinder overlaid the scanned images of the patient's heart.
21. The computer-readable medium of Embodiment 20, wherein the scanned images of the patient's heart are at least one of CT images and MRI images.
22. The computer-readable medium of Embodiment 14, wherein defining an average diameter of at least one cross section around the mitral valve annulus comprises:
   defining a mitral valve plane;
   slicing anatomy above and below the defined mitral valve plane;
   capturing a contour of lumen through each cross-section; and
   extracting an average diameter measurements for each cross-section.
23. The computer-readable medium of Embodiment 22, wherein the average diameter measurements are extracted using an arc method.
24. The computer-readable medium of Embodiment 14, wherein the valve is a pulmonary valve.
Furthermore, equivalents and modifications not described above may also be employed without departing from the scope of the invention, which is defined in the accompanying claims.

## Claims

1. A method of selecting a size for a device to be implanted in a valve of an organ, the method comprising:
acquiring scanned images of the organ;
generating, by a computer, a 3-D model of the organ based on the scanned images;
placing control points in the 3-D model of the organ to define a 3-D valve annulus of the valve of the organ;
taking one or more measurements of relevant anatomical structures based on the 3-D valve annulus; and
selecting the size for the device to be implanted based on the measurements, wherein selecting the size of the device to be implanted comprises:
generating a primitive shape to simulate the device based on the measurements;
virtually placing the primitive shape within the 3-D model of the organ; and
verifying the primitive shape by visualizing, by the computer, the primitive shape overlaid on the scanned images of the organ.

2. The method of claim 1, further comprising verifying and editing the control points on reformatted image views.

3. The method of claim 1 or 2, wherein the primitive shape is a cylinder.

4. The method of any one of claims 1-3, wherein the scanned images are CT or MRI images.

5. The method of any one of claims 1-4, further comprising:
performing, by the computer, a distance mapping between the primitive shape and anatomical structures of the organ; and
visualizing, by the computer, a spacing between the primitive shape the anatomical structures using the distance mapping.

6. The method of any one of claims 1-5, further comprising:
fitting, by the computer, a plane through the 3-D valve annulus;
projecting, by the computer, the 3-D valve annulus onto the plane to define a flattened valve annulus;
measuring a diameter of the flattened valve annulus; and
using the diameter of the flattened valve annulus to size the primitive shape.

7. The method of any one of claims 1-6, wherein taking the one or more measurements comprises:
fitting, by the computer, a plane through the 3-D valve annulus to define a cross-section;
capturing, by the computer, a contour of the lumen of the valve through the cross-section; and
determining an average diameter for the cross-section.

8. The method of claim 7, wherein taking the one or more measurements further comprises:
defining a plurality of cross-sections above and below the plane;
capturing, by the computer, a contour of the lumen through each cross-section; and
determining an average diameter for each cross-section.

9. The method of any one of claims 1-8, wherein taking the one or more measurements comprises calculating, by the computer, a 3-D surface area of the 3-D valve annulus.

10. The method of any one of claims 1-9, wherein a spline is defined by selecting the control points.

11. The method of any one of claims 1-10, further comprising assessing vulnerable anatomical structures based on the one or more measurements.

12. The method of any one of claims 1-11, wherein the 3-D valve annulus is of a mitral valve, wherein the organ is a patient's heart, and further comprising taking one or more additional measurements and using the one or more additional measurements to select the size for the device to be implanted, wherein the one or more additional measurements comprise at least one of
a distance between a first papillary muscle head and a second papillary muscle head;
a distance from at least one papillary muscle head to a best-fit plane through the 3-D valve annulus;
a distance from at least one papillary muscle head to a geometric center of the 3-D valve annulus;
a distance from a ventricular apex of the patient's heart to the geometric center of the 3-D valve annulus; and
a distance from a left atrium roof to the geometric center of the defined 3-D valve annulus.

13. The method of any one of claims 1-11, wherein the 3-D valve annulus is of a pulmonary valve.

14. The method of any one of claims 1-13, wherein the organ is a patient's heart and wherein generating the 3-D model comprises:
calculating, by the computer, based on the scanned images, a 3-D model of a blood volume of the patient's heart; and
reconstructing, by the computer, the patient's heart using the calculated 3-D model of the blood volume.

15. A computer-readable medium having computer-executable instructions stored thereon, which, when executed by a processor of a computing device, cause the computing device to perform the method of any one of claims 1-14.

## Patentansprüche

1. Verfahren zum Auswählen einer Größe für eine Vorrichtung, die in eine Klappe eines Organs implantiert werden soll, das Verfahren umfassend:
Erfassen gescannter Bilder des Organs;
Erzeugen, durch einen Computer, eines 3-D-Modells des Organs basierend auf den gescannten Bildern;
Platzieren von Kontrollpunkten in dem 3-D-Modell des Organs, um einen 3-D-Klappenanulus der Klappe des Organs zu definieren;
Vornehmen einer oder mehrerer Messungen relevanter anatomischer Strukturen basierend auf dem 3-D-Klappenanulus; und
Auswählen der Größe für die zu implantierende Vorrichtung basierend auf den Messungen, wobei das Auswählen der Größe der zu implantierenden Vorrichtung umfasst:
Erzeugen einer Grundform, um die Vorrichtung basierend auf den Messungen zu simulieren;
virtuelles Platzieren der Grundform innerhalb des 3-D-Modells des Organs; und
Verifizieren der Grundform durch Visualisieren, durch den Computer, der Grundform, die den gescannten Bildern des Organs überlagert ist.

2. Verfahren nach Anspruch 1, ferner umfassend ein Verifizieren und Bearbeiten der Kontrollpunkte auf umformatierten Bildansichten.

3. Verfahren nach Anspruch 1 oder 2, wobei die Grundform ein Zylinder ist.

4. Verfahren nach einem der Ansprüche 1 bis 3, wobei die gescannten Bilder CT- oder MRT-Bilder sind.

5. Verfahren nach einem der Ansprüche 1 bis 4, ferner umfassend:
Durchführen, durch den Computer, einer Abstandsabbildung zwischen der Grundform und anatomischen Strukturen des Organs; und
Visualisieren, durch den Computer, einer Beabstandung zwischen der Grundform und den anatomischen Strukturen unter Verwendung der Abstandsabbildung.

6. Verfahren nach einem der Ansprüche 1 bis 5, ferner umfassend:
Anpassen, durch den Computer, einer Ebene durch den 3-D-Klappenring hindurch;
Projizieren, durch den Computer, des 3-D-Klappenanulus auf die Ebene, um einen abgeflachten Klappenanulus zu definieren;
Messen eines Durchmessers des abgeflachten Klappenanulus; und
Verwenden des Durchmessers des abgeflachten Klappenanulus, um die Größe der Grundform festzulegen.

7. Verfahren nach einem der Ansprüche 1 bis 6, wobei das Vornehmen der einen oder der mehreren Messungen umfasst:
Einpassen, durch den Computer, einer Ebene durch den 3-D-Klappenanulus hindurch, um einen Querschnitt zu definieren;
Erfassen, durch den Computer, einer Kontur des Lumens der Klappe durch den Querschnitt hindurch; und
Bestimmen eines durchschnittlichen Durchmessers für den Querschnitt.

8. Verfahren nach Anspruch 7, wobei das Vornehmen der einen oder der mehreren Messungen ferner umfasst:
Definieren einer Vielzahl von Querschnitten oberhalb und unterhalb der Ebene;
Erfassen, durch den Computer, einer Kontur des Lumens durch jeden Querschnitt hindurch; und
Bestimmen eines durchschnittlichen Durchmessers für jeden Querschnitt.

9. Verfahren nach einem der Ansprüche 1 bis 8, wobei das Vornehmen der einen oder der mehreren Messungen ein Berechnen, durch den Computer, einer 3-D-Oberfläche des 3-D-Klappenanulus umfasst.

10. Verfahren nach einem der Ansprüche 1 bis 9, wobei ein Spline durch Auswählen der Kontrollpunkte definiert wird.

11. Verfahren nach einem der Ansprüche 1 bis 10, ferner umfassend ein Beurteilen gefährdeter anatomischer Strukturen basierend auf der einen oder den mehreren Messungen.

12. Verfahren nach einem der Ansprüche 1 bis 11, wobei der 3-D-Klappenanulus von einer Mitralklappe ist, wobei das Organ ein Herz eines Patienten ist, und ferner umfassend das Vornehmen einer oder mehrerer zusätzlicher Messungen und das Verwenden der einen oder der mehreren zusätzlichen Messungen, um die Größe für die zu implantierende Vorrichtung auszuwählen, wobei die eine oder die mehreren zusätzlichen Messungen mindestens eines umfassen von
einem Abstand zwischen einem ersten Papillarmuskelkopf und einem zweiten Papillarmuskelkopf;
einem Abstand von mindestens einem Papillarmuskelkopf zu einer am besten passenden Ebene durch den 3-D-Klappenanulus hindurch;
einem Abstand von mindestens einem Papillarmuskelkopf zu einer geometrischen Mitte des 3-D-Klappenanulus;
einem Abstand von einem ventrikulären Scheitelpunkt des Herzens des Patienten zu der geometrischen Mitte des 3-D-Klappenanulus; und
einem Abstand von einem linken Vorhofdach zu der geometrischen Mitte des definierten 3-D-Klappenanulus.

13. Verfahren nach einem der Ansprüche 1 bis 11, wobei der 3-D-Klappenanulus von einer Pulmonalklappe ist.

14. Verfahren nach einem der Ansprüche 1 bis 13, wobei das Organ ein Herz des Patienten ist und wobei das Erzeugen des 3-D-Modells umfasst:
Berechnen, durch den Computer, basierend auf den gescannten Bildern, eines 3-D-Modells eines Blutvolumens des Herzens des Patienten; und
Rekonstruieren, durch den Computer, des Herzens des Patienten unter Verwendung des berechneten 3-D-Modells des Blutvolumens.

15. Computerlesbares Medium, das computerausführbare Anweisungen aufweist, die darauf gespeichert sind, die, wenn sie durch einen Prozessor einer Rechenvorrichtung ausgeführt werden, die Rechenvorrichtung veranlassen, das Verfahren nach einem der Ansprüche 1 bis 14 durchzuführen.

## Revendications

1. Procédé de sélection d'une taille pour un dispositif à implanter dans une valve d'un organe, le procédé comprenant :
l'acquisition d'images numérisées de l'organe ;
la génération, par un ordinateur, d'un modèle 3D de l'organe à partir des images numérisées ;
le placement de points de contrôle dans le modèle 3D de l'organe pour définir un anneau de valve 3D de la valve de l'organe ;
la prise d'une ou de plusieurs mesures de structures anatomiques pertinentes sur la base de l'anneau de valve 3D ; et
la sélection de la taille pour le dispositif à implanter sur la base des mesures, dans lequel la sélection de la taille du dispositif à implanter comprend :
la génération d'une forme primitive pour simuler le dispositif sur la base des mesures ;
le placement virtuel de la forme primitive dans le modèle 3D de l'organe ; et
la vérification de la forme primitive en visualisant, par l'ordinateur, la forme primitive superposée aux images numérisées de l'organe.

2. Procédé selon la revendication 1, comprenant en outre la vérification et l'édition des points de contrôle sur des vues d'image reformatées.

3. Procédé selon la revendication 1 ou 2, dans lequel la forme primitive est un cylindre.

4. Procédé selon l'une quelconque des revendications 1 à 3, dans lequel les images numérisées sont des images CT ou IRM.

5. Procédé selon l'une quelconque des revendications 1 à 4, comprenant en outre :
la réalisation, par l'ordinateur, d'une cartographie de distance entre la forme primitive et les structures anatomiques de l'organe ; et
la visualisation, par l'ordinateur, d'un espacement entre la forme primitive et les structures anatomiques à l'aide de la cartographie de distance.

6. Procédé selon l'une quelconque des revendications 1 à 5, comprenant en outre :
l'ajustement, par l'ordinateur, d'un plan à travers l'anneau de valve 3D :
la projection, par l'ordinateur, de l'anneau de valve 3D sur le plan pour définir un anneau de valve aplati ;
la mesure d'un diamètre de l'anneau de valve aplati ; et
l'utilisation du diamètre de l'anneau de valve aplati pour dimensionner la forme primitive.

7. Procédé selon l'une quelconque des revendications 1 à 6, dans lequel la prise de la ou des mesures comprend :
l'ajustement, par l'ordinateur, d'un plan à travers l'anneau de valve 3D pour définir une section transversale ;
la capture, par l'ordinateur, d'un contour de la lumière de la valve à travers la section transversale ; et
la détermination d'un diamètre moyen pour la section transversale.

8. Procédé selon la revendication 7, dans lequel la prise de la ou des mesures comprend en outre :
la définition d'une pluralité de sections transversales au-dessus et en-dessous du plan ;
la capture, par l'ordinateur, d'un contour de lumière à travers chaque section transversale ; et
la détermination d'un diamètre moyen pour chaque section transversale.

9. Procédé selon l'une quelconque des revendications 1 à 8, dans lequel la prise de la ou des mesures comprend le calcul, par l'ordinateur, d'une aire 3D de l'anneau de valve 3D.

10. Procédé selon l'une quelconque des revendications 1 à 9, dans lequel une spline est définie en sélectionnant les points de contrôle.

11. Procédé selon l'une quelconque des revendications 1 à 10, comprenant en outre l'évaluation de structures anatomiques vulnérables sur la base de la ou des mesures.

12. Procédé selon l'une quelconque des revendications 1 à 11, dans lequel l'anneau de valve 3D est d'une valve mitrale, dans lequel l'organe est le cœur d'un patient, et comprenant en outre la prise d'une ou de plusieurs mesures supplémentaires et l'utilisation de la ou des mesures supplémentaires pour sélectionner la taille du dispositif à implanter, dans lequel la ou les mesures supplémentaires comprennent au moins l'une parmi
une distance entre un premier chef musculaire papillaire et une seconde tête de muscle papillaire ;
une distance allant d'au moins un chef musculaire papillaire à un plan d'ajustement optimal de l'anneau de valve 3D défini ;
une distance allant d'au moins un chef musculaire papillaire à un centre géométrique de l'anneau de valve 3D défini ;
une distance allant d'un sommet ventriculaire du cœur du patient au centre géométrique de l'anneau de valve 3D défini ; et
une distance allant du toit de l'oreillette gauche au centre géométrique de l'anneau de valve 3D défini.

13. Procédé selon l'une quelconque des revendications 1 à 11, dans lequel l'anneau de valve 3D est celui d'une valve pulmonaire.

14. Procédé selon l'une quelconque des revendications 1 à 13, dans lequel l'organe est le cœur d'un patient et dans lequel la génération du modèle 3D comprend :
le calcul, par l'ordinateur, sur la base des images numérisées, d'un modèle 3D d'un volume sanguin du cœur du patient ; et
la reconstruction, par l'ordinateur, du cœur du patient à l'aide du modèle 3D calculé du volume sanguin.

15. Support lisible par ordinateur sur lequel sont stockées des instructions exécutables par ordinateur qui, lorsqu'elles sont exécutées par un processeur d'un dispositif informatique, amènent le dispositif informatique à réaliser le procédé selon l'une quelconque des revendications précédentes 1 à 14.
